# FASCICULE DE BREVET EUROPEEN

(11) **EP 3 052 153 B1**
(45) Date de publication et mention de la délivrance du brevet: **03.07.2019**
(21) Numéro de dépôt: 14786353.4
(22) Date de dépôt: 01.10.2014
(51) Int. Cl.: A61L 27/24, C07K 14/78

(54) **PROCEDE DE PREPARATION D'UNE MATRICE DE COLLAGENE FIBRILLE**
VERFAHREN ZUR HERSTELLUNG EINER FIBRILLÄREN KOLLAGENMATRIX
METHOD FOR PREPARING A FIBRILLAR COLLAGEN MATRIX

(30) Priorité: 02.10.2013 FR 1359555
(43) Date de publication de la demande: 10.08.2016
(73) Titulaire: Sorbonne Université, 75006 Paris (FR); Centre National de la Recherche Scientifique, 75794 Paris Cedex 16 (FR)
(72) Inventeur: MOSSER, Gervaise, F-75014 Paris (FR)
(74) Mandataire: Gevers & Orès
(86) Numéro de dépôt international: PCT/IB2014/064990
(87) Numéro de publication internationale: WO 2015/049646

(56) Documents cités:
- EP-A1- 2 966 047
- WO-A2-2007/106812
- FR-A1- 2 944 706
- FR-A1- 2 966 047

## Description

La présente invention a pour objet un procédé de préparation d'une matrice de collagène fibrillé, avantageusement transparente ou composite, apte à être utilisée comme biomatériau, notamment comme substitut cornéen.

Le collagène est la protéine la plus abondante chez les mammifères. C'est une protéine structurale majeure des tissus conjonctifs, dans lesquels il remplit le rôle d'armature.

En effet, les types de collagène les plus abondants s'assemblent sous la forme de fibrilles dont la résistance, à poids égal, surpasse celle de l'acier. Ils sont associés au sein d'édifices supramoléculaires à d'autres types de collagène ainsi qu'à d'autres macromolécules partenaires. La trame de fibrilles de collagène est non seulement responsable de la cohésion des tissus mais aussi de leur architecture spécifique qui leur confère des propriétés mécaniques particulières. Ainsi, l'arrangement architectural des fibrilles de collagène répond de façon optimale aux fonctions des tissus considérés suivant leurs localisations.

Les molécules de collagène sont solubles en milieu acide *in vitro* ; les matériaux à base de collagène sont obtenus à partir de solutions acido-soluble de collagène. De manière générale, une matrice de collagène est obtenue par concentration d'une solution diluée de collagène selon différentes techniques (Wang, Y. et al., Soft Matter 2011, 7, 9659). Les propriétés de la matrice de collagène ainsi obtenue peuvent ensuite être améliorées par des étapes supplémentaires de réticulation ou de compression.

Ratanavaraporn et al. (J Biomater Sci Polym, Ed. 2008; 19(7) : 945-52) décrivent l'effet du type d'acide utilisé pour solubiliser le collagène sur les propriétés physiques et biologiques de matériaux à base de collagène obtenus par lyophilisation et réticulation. Les auteurs limitent leur étude à la comparaison des effets d'une solubilisation du collagène soit par de l'acide acétique soit par de l'acide chlorhydrique.

Lorsque la concentration en collagène est supérieure à 45-100 mg/ml, les molécules s'auto-assemblent spontanément de par leurs propriétés lyotropiques et forment des phases cristal-liquide (N. S. Murthy, Biopolymers, 1984, 23, 1261 ; MM. Giraud-Guille, Biol Cell, 1989, 67, 97-101; G. Mosser et al. Matrix Biol. 2006, 25, 3-13 ; F. Gobeaux et al. Langmuir, 2007, 23, 6411-6417; P. De Sa Peixoto et al. Soft Matter, Vol., 7, 2011, pp. 11203-11210).

De Sa Peixoto et al. (Soft Matter, Vol., 7, 2011, pp. 11203-11210) analysent l'influence de la force ionique et des paramètres du solvant sur l'organisation des phases cristal-liquide de collagène I. Les auteurs évaluent notamment le phénomène de transition du collagène en solution acide de la phase isotrope / phase liquide cristalline en faisant varier le type d'acide et la concentration. Les auteurs évaluent les effets de l'acide chlorhydrique et l'acide acétique séparément.

L'assemblage des molécules de collagène entre elles est traditionnellement désigné sous le terme de « fibrillogenèse ». Les interactions entre molécules seraient dans un premier temps assurées par des liaisons hydrogènes et des interactions électrostatiques. Les mécanismes générant les différentes architectures des fibrilles de collagène *in vivo* sont encore mal connus et plusieurs hypothèses ont été formulées. L'une de ces hypothèses décrit la genèse de ces assemblages comme un processus extracellulaire d'auto-assemblage de triples hélices de collagène, principalement gouverné par des forces physico-chimiques classiques.

*In vitro,* la fibrillogenèse d'une solution de collagène peut être obtenue par plusieurs techniques.

Par exemple, il est possible d'induire la fibrillogenèse du collagène acido-soluble en faisant varier le pH et la force ionique (R.L. Trelstad, et al. 1976, Proc. Natl. Acad. Sci. U.S.A., 73, 4027-4031; J. Robin Harris & Andreas Reiber, Micron, 2007, 38 513-521) ou en modulant la concentration en collagène de la solution (F. Gobeaux et al., J Mol Biol, 2008, 376, 1509-1522).

La fibrillogenèse d'une solution acido-soluble de collagène peut donc être induite par augmentation du pH et, dans le cas d'une solution concentrée, on observe alors une transition sol-gel.

Dans les tissus vivants, plusieurs types d'organisation de macromolécules biologiques sont rencontrés en fonction des propriétés mécaniques requises (Hulmes et al., 2002, Journal of Structural Biology 137, 2-10). Par exemple, une structure en phase cholestérique est observée dans l'os compact et les travées osseuses de l'os spongieux, cette organisation tridimensionnelle du collagène contribue aux propriétés mécaniques de l'os. Elle peut être recréée et stabilisée *in vitro.* De même il est bien connu que l'organisation des fibrilles de collagène est unidirectionnelle au niveau des tendons, tandis qu'elle se présente sous forme de contreplaqué au niveau de la cornée.

De nombreuses techniques permettent l'obtention de matrices de collagène fibrillé présentant des propriétés mécaniques mimant différents tissus.

Ainsi, le laboratoire de l'inventeur est à l'origine du développement de techniques permettant de travailler avec du collagène à des concentrations élevées et ainsi de tirer avantage des propriétés lyotropiques du collagène, en contrôlant la mise en place d'un ordre supramoléculaire lié à la concentration et en stabilisant cette organisation lors d'une transition sol/gel pour aboutir à des matrices fibrillaires denses. Ces techniques ont été déclinées pour obtenir des matériaux ayant chacun des propriétés propres.

La demande WO 2010/004182 concerne un procédé de minéralisation de matrices fibrillaires denses de collagène pour une application dans le domaine des substituts osseux.

La demande WO 2011/151587 présente un procédé d'obtention de matrices fibrillaires denses de collagène obtenues à des concentrations 5 à 60 fois plus élevées que les hydrogels classiques. La concentration en collagène des matériaux est d'environ 5 à environ 1000 mg/ml. Selon leur concentration et leur épaisseur, de tels matériaux sont aptes à être utilisés comme substitut tissulaire, notamment comme renfort de paroi, pour la fabrication de prothèses, comme substitut de tissu mou ou comme matériau de comblement.

La demande WO 2012/052679 présente un procédé d'obtention de matrices fibrillaires transparentes. Le procédé permet de favoriser une croissance longitudinale plutôt que transverse (fibrilles de faible diamètre et longues) et de générer un écartement régulier entre les fibrilles. Les matrices obtenues selon ce procédé sont transparentes.

Une limite commune aux techniques exposées ci-dessus consiste en la difficulté de contrôler les propriétés mécaniques et optiques des matrices de collagène.

Particulièrement, concernant la préparation de matrices transparentes, il est connu de l'homme du métier que la transparence du matériau diminue quand la densité de collagène du matériau augmente. Il reste donc difficile de synthétiser des matrices de collagène transparentes présentant à la fois des propriétés mécaniques et des propriétés optiques optimales.

En outre, la plupart des techniques exposées ci-dessus ne permettent pas de contrôler facilement la forme des matrices de collagène. Aussi existe-t-il un besoin de mettre au point un procédé simple qui permette de mouler les matrices de collagène tout en contrôlant leurs propriétés mécaniques et optiques, ceci notamment dans le cas de matrices de collagène transparentes.

La présente invention découle de la mise en évidence que les propriétés mécaniques et optiques des matrices de collagène peuvent être modulées par l'utilisation de solutions de collagène comprenant au moins un acide fort et un acide faible.

Les inventeurs ont mis au point procédé qui permet l'obtention de matrices de collagène présentant à la fois des caractéristiques mécaniques et optiques d'un grand intérêt pour la fabrication de tissus ou d'organes artificiels, notamment comme substitut de cornée ou comme support pour la croissance de cellules. Ce nouveau procédé de fabrication permet plus particulièrement, de synthétiser des matrices de collagène fibrillé dont la transparence peut être modulée d'entièrement transparente à entièrement opaque ou encore de synthétiser des matrices de collagène fibrillé composite, comprenant des zones opaques et des zones transparentes.

Aussi, la présente invention a pour objet un procédé de préparation d'une matrice de collagène fibrillé comprenant une étape de fibrillogenèse d'un collagène, en phase cristalline liquide ou non, ladite étape étant réalisée dans un milieu comprenant au moins un acide fort et un acide faible.

Sans vouloir être liés à une théorie particulière, les inventeurs pensent que la présence d'une combinaison d'un acide fort et d'un acide faible au contact du collagène retentit sur la croissance transverse des fibrilles et leur espacement lors de l'étape de fibrillogenèse.

Au sens de la présente invention on entend par collagène fibrillé un collagène présentant une agrégation non aléatoire des molécules de collagène et une période D d'environ 67 nm. Au sens de l'invention, le collagène utilisé est un collagène qui peut s'organiser en fibrilles, comme le collagène de type I, II, III et/ou V. Avantageusement, le collagène utilisé est de type I, éventuellement associé à d'autres types de collagènes.

La notion d'acide fort et d'acide faible est bien connue de l'homme du métier. Au sens de l'invention et en référence aux travaux de Brønsted-Lowry, un acide est dit « fort » lorsque, en solution aqueuse, il se dissocie selon une réaction quasi-totale dans l'eau quelle que soit la concentration apportée. Les acides forts peuvent être choisis, par exemple, parmi l'acide chlorhydrique ; l'acide sulfurique ; l'acide perchlorique ; l'acide fluorhydrique ; l'acide iodhydrique ; l'acide chlorique ; l'acide permanganique ; l'acide manganique. Un acide est dit «faible» lorsque, en solution aqueuse, il se dissocie avec l'eau selon une réaction qui n'est pas totale. Les acides faibles peuvent être choisis, par exemple, parmi l'acide acétique, l'acide chloro-, dichloro-, trichloro-acétique ; l'acide bromoacétique ; l'acide trifluoro-acétique ; l'acide propionique ; l'acide ascorbique ; l'acide valérique ; l'acide 2-, 4-, 5-bromovalérique ; l'acide lactique ; l'acide citrique ; l'acide salicylique ; l'acide acétylsalicylique ; l'acide formique, les acides de la famille des acides uroniques, de préférence l'acide glucuronique ; l'acide galacturonique

Dans un mode de réalisation de l'invention, avant fibrillogenèse, le collagène est sous une forme cristal liquide (organisé soit en phase nématique-torsadé, soit en phase nématique-contreplaqué, smectique ou hexagonal, soit toute autre type d'organisation cristal liquide) ou isotrope.

Conformément à l'invention, la matrice de collagène fibrillé peut être réalisée en mettant à profit les techniques connues de l'homme du métier.

Dans un mode de réalisation avantageux de l'invention, le procédé de préparation d'une matrice de collagène fibrillé est caractérisé en ce qu'il comprend les étapes suivantes :
(a) la préparation d'une solution aqueuse acide comprenant- du collagène sous forme de monomères à une concentration comprise entre 0,1 et 10 mg/ml, avantageusement comprise entre 0,3 et 5 mg/ml, et- au moins un acide fort présent en une concentration de 0,01 à 10 mM et un acide faible présent en une concentration comprise entre 0,1 et 500 mM,
(b) la concentration de la solution obtenue en (a) jusqu'à une concentration en collagène comprise entre 10 et 250 mg/ml;
(c) la formation des fibrilles par traitement de la solution de collagène obtenue à l'étape (b) par une phase gazeuse basique ou par une phase liquide basique.

Conformément à l'invention, la solution de collagène de l'étape a) est obtenue par des techniques connues de l'homme du métier à partir d'une solution initiale de collagène naturel ou recombinant et de solutions d'acide fort et d'acide faible. La concentration en collagène d'une telle solution est comprise entre 0,1 et 10 mg/ml, avantageusement comprise entre 0,3 et 5 mg/ml. La solution de collagène selon l'étape a) présente un pH inférieur ou égal à 4, de préférence compris entre 2 et 4. Dans ces solutions, le collagène est généralement sous forme de monomères (triples hélices) et éventuellement sous forme de nanofibrilles.

Le collagène mis en oeuvre dans l'étape a) est un collagène sous forme de monomères.

Selon un mode de réalisation de l'invention, la solution de l'étape a) est préparée par la technique de dialyse. L'homme du métier peut aisément déterminer les paramètres de dialyse (type de membrane, concentrations en solutés des liquides de contre-dialyse) en s'appuyant notamment sur les lois régissant les phénomènes de transport à travers les membranes. Ainsi, la solution de l'étape a) peut être obtenue par dialyse d'une solution acide de collagène comprenant un premier acide, par exemple un acide faible, contre une solution de contre-dialyse comprenant un deuxième acide, par exemple un acide fort, ou un mélange d'acides fort et faible.

Dans la solution aqueuse acide de l'étape a),
- l'acide faible peut être présent à une concentration comprise entre 0,1 et 500 mM, avantageusement de 0,2 à 200 mM, et préférentiellement de 2 à 125 mM ; et
- l'acide fort peut être présent en une concentration de 0,01 à 10 mM, avantageusement de 0,1 à 3 mM et préférentiellement de 0,2 à 2,5 mM.

Avantageusement, l'acide fort utilisé est l'acide chlorhydrique et l'acide faible utilisé est l'acide acétique.

Dans un mode de réalisation avantageux de l'invention, la solution aqueuse acide de collagène de l'étape a) contient de l'acide acétique à une concentration comprise entre 0,1 et 500 mM, de préférence de 0,2 à 200 mM et préférentiellement de 2 à 125 mM et de l'acide chlorhydrique à une concentration de 0,01 à 10 mM, de préférence de 0,1 à 3 mM et préférentiellement de 0,2 à 2,5 mM.

De manière conforme à l'invention, l'étape de concentration de la solution de collagène (b) est réalisée par des techniques connues de l'homme du métier.

L'étape de concentration peut notamment être effectuée par une quelconque technique appropriée utilisant une membrane sélective, par exemple, par la filtration-centrifugation, par l'ultrafiltration, par évaporation rapide sous cryo-centrifugation ou par l'osmose inverse. Comme on le sait, les techniques de filtration membranaire utilisent des membranes sélectives pour séparer des espèces moléculaires selon leur poids moléculaire. De telles membranes sont caractérisées par leur poids moléculaire de séparation ou par la taille des pores. Ainsi, les membranes sélectives utilisées pour une concentration par ultrafiltration présentent des pores dont la taille est comprise entre 0,001 et 0,1 µm environ. Les membranes sélectives utilisées pour une concentration par osmose inverse se présentent sous la forme de membranes empilées, pouvant présenter une porosité très faible ne laissant alors passer que le solvant et arrêtant les ions. Selon un mode avantageux de l'invention, la concentration de la solution peut être réalisée par la technique de filtration-centrifugation. A cet effet, on peut par exemple utiliser des membranes dont le poids moléculaire de séparation est de 100 kDa, 50 kDa, 40 kDa, 30 kDa, 20kDa, 10 kDa, 3 kDa. Préférentiellement, des membranes de 30 kDa ou des membranes dont la taille est comprise entre 4 et 5 nm environ peuvent être utilisées. L'homme du métier peut facilement déterminer les paramètres de centrifugation en fonction des caractéristiques du concentrateur et de la concentration en collagène attendue. La concentration en collagène à l'issue de l'étape b) peut être comprise entre 10 et 250 mg/ml, préférentiellement entre 30 et 200 mg/ml, et avantageusement entre 40 et 120 mg/ml.

L'étape de concentration (b) permet d'avoir, avant la fibrillogenèse, un collagène en solution dense et avantageusement sous forme cristal liquide avec une organisation ordonnée et hiérarchique des molécules de collagène. L'obtention d'une phase cristal liquide organisée contribue également à l'optimisation des propriétés mécaniques et optiques des matrices de collagène.

Lorsqu'un matériau est à utiliser comme substitut de tissus ou d'organes, généralement plus ledit matériau mime le tissu ou l'organe où l'implantation doit avoir lieu, mieux le matériau s'implantera et permettra aux cellules de l'hôte de se l'approprier pour régénérer un tissus sain. Ainsi, mimer un tissu ou un organe signifie mimer ou imiter ses constituants biochimiques, son organisation tri-dimensionnelle et ses propriétés mécaniques. Les phases cristal liquides permettent d'obtenir une organisation tridimensionnelle qui mime celle trouvée dans les tissus et organes du vivant.

A l'issue de l'étape (b), le collagène est donc sous une forme cristal liquide, organisé, par exemple, soit en phase nématique-torsadé (cholestérique), soit en phase nématique-contreplaqué, smectique ou hexagonal, soit toute autre type d'organisation cristal liquide.

Selon un mode de réalisation particulier de l'invention, le procédé peut comporter, en outre, après l'étape de concentration (b) et avant l'étape de formation des fibrilles ou fibrillogenèse (c), une étape de mise en forme de la solution de collagène. Au sens de la présente invention, on entend par étape de mise en forme toute étape au cours de laquelle la solution de collagène obtenue à l'issue de l'étape b) peut notamment être placée dans un moule. Dans un mode particulier de l'invention, au moins deux solutions de collagène obtenues à l'issue de l'étape b) comportant des concentrations différentes en collagène et/ou en acides forts et/ou en acide faibles peuvent être juxtaposées dans un même moule. Alternativement, les solutions de collagène peuvent mises en forme par couche successives. Ainsi, l'étape de mise en forme de la solution de collagène peut être une étape de moulage. Dans le procédé de l'invention, l'organisation cristal liquide des solutions de collagène peut être maintenue lors de l'étape de mise en forme ou restaurée après celle-ci par une étape de relaxation. Avantageusement, l'organisation cristal liquide des solutions de collagène est maintenue lors de l'étape de mise en forme.

Selon un mode particulier de l'invention, le procédé peut donc comporter une étape de moulage de différentes solutions de collagène obtenues à l'issue de l'étape b). Après l'étape de formation des fibrilles ou fibrillogenèse (c), une telle juxtaposition de solutions de collagène dans un moule mène à l'obtention d'une matrice comportant des zones dont les propriétés sont différentes, en termes de densité de collagène, de transparence, et/ou de propriétés mécaniques. Cette possibilité peut notamment être mise à profit en vue d'obtenir un substitut de tissu comportant une aire mimant les propriétés de la sclérotique et une aire mimant les propriétés de la cornée. Ainsi, en mettant en oeuvre le procédé selon la présente invention, les inventeurs ont pu obtenir une matrice de collagène composite, comprenant une zone centrale transparente à la lumière et une zone périphérique opaque à la lumière. Une telle matrice de collagène peut être utile comme substitut cornéen. Dans un autre contexte, cette possibilité peut notamment être mise à profit en vue d'obtenir un substitut comportant une aire minéralisée et une autre non-minéralisée mimant les propriétés d'une jonction os-tendon.

De manière conforme à l'invention, la formation des fibrilles (ou fibrillogenèse) dans la solution de collagène à l'étape (c) est réalisée par augmentation du pH de la solution concentrée de collagène obtenue à l'étape (b) selon des techniques connues de l'homme du métier. À l'issue de l'étape c), la matrice de collagène présente un pH compris entre 5 et 13.

Ainsi, la formation des fibrilles (ou fibrillogenèse) dans la solution de collagène à l'étape (c) peut être réalisée par traitement de la solution de collagène obtenue à l'étape (b) par une phase gazeuse ou un liquide.

Une manière pratique d'induire la fibrillogenèse est de soumettre une solution acide de collagène, éventuellement mise en forme, à une phase gazeuse constituée de vapeurs d'ammoniac. Les vapeurs, en se solubilisant dans le solvant, induisent une augmentation de pH de la solution de collagène. Ainsi, selon un premier mode de réalisation, une solution concentrée de collagène obtenue à l'issue de l'étape b), éventuellement mise en forme, peut être placée pendant 15 heures environ dans une enceinte contenant de la vapeur d'ammoniac. Une autre technique de fibrillogenèse de solutions de collagène consiste à dialyser un petit volume de solution acide de collagène contre un grand volume d'une solution aqueuse tamponnée à un pH compris entre 5 et 13. Les solutions de collagène présentant une concentration en collagène au-delà de 40 mg/ml constituent des solutions extrêmement visqueuses, voire de véritables gels physiques. Dans ces conditions, les solutions de collagène concentrées peuvent être fibrillés par immersion directe dans une solution aqueuse de pH compris entre 5 et 13 pendant au moins 48 heures à 20°C. On peut citer à titre d'exemple de solution aqueuse de pH compris entre 5 et 13 le tampon phosphate salin ou l'eau ammoniacale.

L'étape de fibrillogenèse par vapeur d'ammoniac permet de conserver la concentration de la solution et l'organisation moléculaire mise en place en phase liquide (étape (b)). Il en résulte alors une phase cristal liquide stabilisée dans laquelle les organisations mises en place en phase liquide sont conservées mais où l'entité organisée n'est plus la molécule de collagène mais la fibrille de collagène.

Selon un mode particulier de l'invention, le procédé peut comporter, en outre, une étape de relaxation après l'étape de mise en forme de la solution de collagène (b) et avant l'étape de formation des fibrilles ou fibrillogenèse (c). Cette période peut favoriser la relaxation du cisaillement induit lors du moulage de la solution de collagène. Cette étape peut permettre de retrouver l'organisation de la phase cristal liquide avant moulage de la solution de collagène. Dans le cas d'une mise en forme par juxtaposition de différentes solutions de collagène dans un moule, cette étape peut favoriser une jonction entre les différentes solutions de collagène. Cette période de relaxation peut varier de 12 heures à 1 semaine, en fonction de la viscosité de la solution.

Selon un mode particulier de l'invention, le procédé peut comporter, en outre, une étape de mûrissement après l'étape de formation des fibrilles ou fibrillogenèse (c). Cette période de mûrissement varie de quelques jours à 2 mois, en fonction de la force ionique, de la température et du pH. Avantageusement l'étape de mûrissement dure de 3 jours à 2 mois, avantageusement de 7 à 15 jours. Une telle période de mûrissement peut favoriser une croissance longitudinale des fibrilles et une augmentation de la transparence.

Le procédé de l'invention permet de préparer des matrices de collagène fibrillé dont la transparence peut être modulée d'entièrement transparente à entièrement opaque. Selon un mode particulier de l'invention, le procédé permet aussi d'obtenir des matrices de collagène fibrillé composite, comprenant des zones opaques et des zones transparentes. Le procédé permet également d'obtenir des matrices de collagène moulées à façon. Ainsi, la présente invention a pour objet un procédé de préparation d'une matrice de collagène fibrillé transparente comprenant les étapes suivantes :
(a) la préparation d'une solution aqueuse acide comprenant :
   - du collagène, avantageusement de type I, éventuellement associé à d'autres types de collagènes, à une concentration comprise entre 0,1 et 10 mg/ml, avantageusement comprise entre 0,3 et 5 mg/ml, et
   - au moins de l'acide acétique et de l'acide chlorhydrique.
(b) la concentration de la solution obtenue en (a) par filtration-centrifugation jusqu'à une concentration en collagène comprise entre 10 et 250 mg/ml, avantageusement entre 30 et 200 mg/ml, encore plus avantageusement entre 40 et 120 mg/ml ;
(c) la mise en forme de la solution de collagène obtenue en (b) ;
(d) la relaxation de la solution de collagène à l'issue de l'étape (c) ;
(e) la formation des fibrilles par traitement de la solution de collagène obtenue à l'étape (d) avec une phase gazeuse basique ;
(f) le mûrissement des fibrilles obtenues à l'étape (e) jusqu'à obtention d'une matrice transparente.

Le collagène utilisé dans ce procédé est un collagène qui peut s'organiser en fibrilles, comme le collagène de type I, II, III et/ou V. Avantageusement, le collagène utilisé est de type I, éventuellement associé à d'autres types de collagènes.

L'invention a pour but de proposer des matrices de collagène fibrillé denses transparentes. Au sens de l'invention, une matrice de collagène est dense lorsque sa densité en collagène est égale ou supérieure à 40 mg/ml, avantageusement 90 mg/ml. Au sens de la présente invention, une matrice transparente de collagène correspond à une matrice de collagène qui transmet au moins 80% de la lumière blanche. Dans certains cas, une matrice de collagène ou une portion de matrice de collagène « transparente » correspond à un matériau ou une portion de matériau dont la transmission de la lumière blanche est équivalente à celle d'une cornée saine, c'est-à-dire dont la transmission de la lumière blanche est d'au moins 90% et la dispersion inférieure à 3%.

En mettant en oeuvre le procédé selon l'invention, les inventeurs ont pu obtenir, par exemple, une matrice de collagène dont la transparence est de 97% pour une densité de collagène de 90 mg/ml ; une matrice de collagène dont la transparence est de 81% pour une densité de collagène de 120 mg/ml.

L'invention a également pour but de proposer des matrices de collagène fibrillé composites comprenant des zones opaques et des zones transparentes, c'est-à-dire au moins une zone qui transmet moins de 80% de la lumière blanche (zones opaques) et au moins une zone transparente qui transmet au moins 80% de la lumière blanche telle que décrites ci-dessus.

Au sens de la présente invention, on entend par matrice de collagène stable, une matrice qui ne présente pas d'évolution de la taille des fibrilles dans des conditions physiologiques (37°C, haute force ionique, etc.) et/ou en présence de cellules. Ainsi les matrices selon l'invention, ne deviennent pas opaques lorsqu'on les met en contact avec un milieu ayant une forte force ionique, notamment un milieu de culture de cellule.

Dans les matrices de collagène selon l'invention, le collagène peut être sous forme de fibrilles bien individualisées et relativement équidistantes et organisées ou sous forme d'agrégats de fibrilles espacés les uns des autres par des fibrilles fines réticulées et imposant une inter-distance relativement régulière.

Le diamètre des fibrilles de collagène dans les matrices transparentes synthétisées peut être entre 6 et 30 nm, bornes incluses. Dans un mode de réalisation avantageux de l'invention, le diamètre desdites fibrilles est de 25 nm. Les fibrilles élémentaires peuvent également s'accoler ou se regrouper en fibres plus grosses (ou agrégats de fibrilles) de 0,2 à 0,5 µm de diamètre (diamètre du coeur de l'agrégat de fibrilles) pour donner des matrices translucides à opaques.

Les matrices de collagène fibrillé selon l'invention peuvent être utilisées pour de nombreuses applications, incluant, mais pas limitées à, des applications cliniques, thérapeutiques, prophylactiques, cosmétiques ainsi qu'en recherche fondamentale. Les matrices de collagène issues du procédé selon l'invention peuvent être utilisées en tant que substitut de tissus ou d'organes d'un mammifère, de préférence humain. L'invention a donc pour objet, l'utilisation d'une matrice de collagène pour la fabrication d'un tissu ou d'un organe artificiel. Ainsi, des matrices de collagène fibrillé transparentes et/ou composites selon l'invention peuvent être utilisées pour fabriquer un substitut de cornée. Plus particulièrement, des matrices de collagène fibrillé comprenant des zones transparentes et des zones opaques, autrement dit des matrices composites, peuvent être utilisées pour la fabrication de substituts de cornée-sclère. Des matrices de collagène fibrillé comprenant des zones minéralisées et des zones non-minéralisées, telles que décrites ci-dessous, peuvent être utilisées pour la fabrication de substituts os-tendon.

L'invention a également pour objet l'utilisation d'une matrice de collagène fibrillé transparente dense ou d'une matrice de collagène fibrillé composite pour la fabrication de tissus ou d'organes artificiels, notamment comme substitut de cornée, comme support pour la croissance de cellules, notamment pour leur analyse comportementale, ou comme support pour l'étude de procédés de bio-minéralisation. Les matrices de collagène selon l'invention présentent une cytotoxicité faible voire absente car elles peuvent être obtenues selon un procédé n'impliquant pas d'espèces chimique toxiques. Les matrices de collagène selon l'invention constituent un milieu favorable à la croissance de cellules ou de nerfs.

Un des objets de l'invention concerne un substitut de cornée comprenant une matrice de collagène, de préférence transparente, composite ou non, obtenue selon le procédé de l'invention.

Les matrices de collagène peuvent être utilisées comme implants ophtalmiques. Les matrices de collagène peuvent être biodégradables, c'est-à-dire présenter une résorption lente et progressive. Ainsi, les matrices de collagène peuvent être utilisées en tant qu'implants biodégradables pour renforcer ou combler un tissu, par exemple un tissu ophtalmique.

Les matrices de collagène de la présente invention peuvent être particulièrement adaptées pour une utilisation dans des dispositifs ophtalmiques, notamment parce qu'elles (i) sont façonnables pour former un matériau avec une puissance optique acceptable, (ii) peuvent être optiquement claires ou transparentes ; (iii) constituent un milieu favorable à l'épithélialisation de la surface antérieure.

La matrice de collagène peut être réalisée de façon à permettre la diffusion des gaz et/ou des nutriments. Ces nutriments peuvent inclure le glucose et/ou des facteurs ou agents de croissance ou de différenciation des cellules, ou toute molécule d'intérêt. Un tel échange peut être comparable ou supérieur à celle d'une cornée humaine saine. La perméabilité du matériau aux éléments d'intérêts peut être surveillée à l'aide de techniques connues de l'homme du métier.

Le procédé de l'invention peut permettre de préparer des matrices de collagène fibrillé minéralisées. Ainsi, l'invention a, en outre, pour objet un procédé d'obtention de matrices minéralisées ainsi que des matrices de collagène fibrillé comprenant des zones minéralisées et des zones non-minéralisées.

De telles matrices comprennent une phase minérale synthétique, qui peut par exemple consister en de l'hydroxyapatite synthétique. Des matrices comprenant une phase minérale peuvent être obtenues selon des techniques connues de l'homme du métier, notamment par précipitation de précurseurs de phase minérale au sein de la matrice de collagène. Au sens de l'invention, des précurseurs de phase minérale peuvent être des ions calcium, des ions phosphate comme l'ion phosphate PO₄³⁻ ; l'ion hydrogénophosphate HPO₄²⁻ ; l'ion dihydrogénophosphate H₂PO⁴⁻ ; ou des ions carbonate comme l'ion carbonate CO₃²⁻, l'ion hydrogénocarbonate HCO₃⁻.

Selon un mode de réalisation particulier qui ne fait pas partie de le présente invention, le procédé peut comporter avant l'étape de concentration b), une étape de dialyse contre une solution aqueuse de pH compris entre 2 et 4 comprenant un acide faible et un acide fort, et des précurseurs de phase minérale. Dans ce mode de réalisation de l'invention, la solution comprend au moins des ions calcium et des ions phosphate.

Selon un autre mode de réalisation particulier qui ne fait pas partie de le présente invention, le procédé peut comporter après l'étape de concentration b) et avant l'étape de mise en forme c), une étape de mise en contact de la solution de collagène (Solution 1) avec une solution aqueuse de pH compris entre 2 et 4 comprenant un acide faible et un acide fort, et des précurseurs de phase minérale (Solution 2). Dans ce mode de réalisation de l'invention, la solution (Solution 2) comprend au moins des ions calcium et des ions phosphate. Dans ce mode de réalisation, la mise en contact entre les Solutions 1 et 2 peut avantageusement être effectuée par la technique filtration-centrifugation. Une solution aqueuse de pH compris entre 2 et 4 comprenant un acide faible et un acide fort, et des précurseurs de phase minérale (Solution 2) est placée au-dessus de la solution acide de collagène (Solution 1) obtenue à l'issue de l'étape b) dans le même tube de centrifugation-filtration. Une centrifugation à basse vitesse (4500 tr/min ou rpm) permet alors d'échanger le solvant de la Solution 1 par celui de la Solution 2 tout en maintenant dans la Solution 1, les concentrations en acide fort, en acide faible et en collagène telle qu'obtenue à l'issue de l'étape b). La centrifugation permet également à la solution de collagène (Solution 1) d'intégrer les ions précurseurs de la minéralisation. Avantageusement, la solution contenant les précurseurs de la minéralisation présente une concentration en précurseur de phosphate, par exemple NaH₂PO₄, inférieure à la limite de solubilité, de préférence de 1,5 à 900 mM, plus particulièrement de 66 à 330 mM ; la concentration en précurseur de calcium, par exemple CaCl₂, est inférieure à la limite de solubilité, de préférence de 2,5 mM à 1,5 M, plus particulièrement de 10 à 550 mM. Encore plus avantageusement, les quantités de précurseurs sont telles que le rapport molaire Ca/P est entre 1,5 et 1,8, de préférence de l'ordre de 1,67. Selon ce mode de mise en oeuvre de l'invention, la précipitation des précurseurs de la phase minérale est réalisée par augmentation du pH de la solution de collagène dans laquelle les précurseurs de phase minérale ont diffusés. Ainsi, la précipitation des précurseurs de la phase minérale peut être réalisée lors de l'étape c), c'est-à-dire de façon concomitante avec la fibrillogenèse par augmentation de pH.

D'autres avantages et caractéristiques de la présente invention pourront encore apparaître à la lecture des exemples ci-dessous donnés à titre illustratif et les figures annexées :
- La Figure 1 représente des exemples de combinaisons de concentrations en acide acétique et en acide chlorhydrique utilisées pour une solution de collagène à concentration initiale de 3,16 mg/ml. Le tableau indique les concentrations en acides et le pH final de la solution de dialyse. Chaque combinaison est indexée par un numéro qui sera utilisé dans les autres figures et dans la suite de l'exposé.

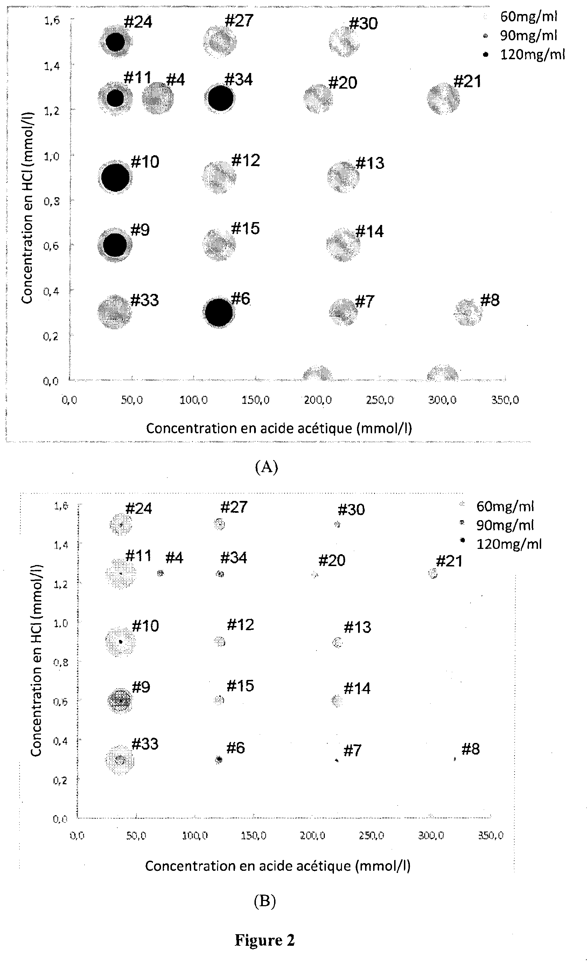

- La Figure 2 représente la transparence (A) et l'inverse du coefficient d'absorption 1/*α_{c}* (B) des matrices de collagène en fonction des conditions physicochimiques répertoriées par leur indexe indiqué en Figure 1. Les rayons des cercles sont proportionnels aux valeurs mesurées. La transparence décroit avec l'augmentation de la concentration en collagène.

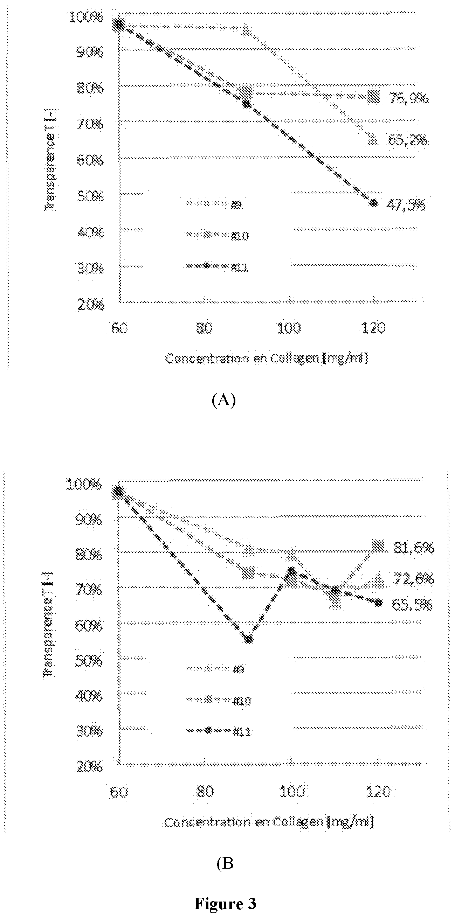

- La Figure 3 représente le pourcentage de chute de la transparence moyenne mesurée entre 530 nm et 580 nm (en ordonnée) en fonction de la concentration en collagène (en abscisse) pour les conditions #9, #10 et #11. Les échantillons obtenus selon les procédures 1 et 2 sont respectivement représentés en (A) et (B).

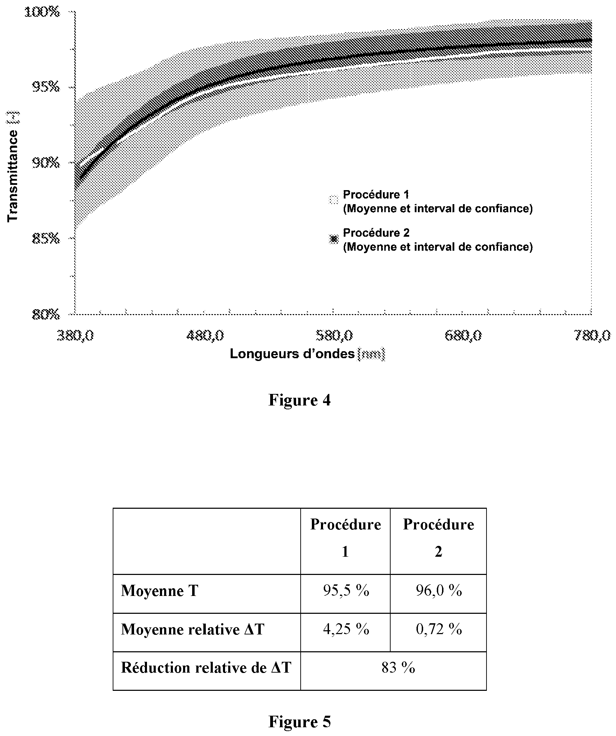

- La Figure 4 représente la moyenne de la transparence et les intervalles de confiance 2σ pour 4 échantillons de condition #11 à 60 mg/ml préparé selon la procédure 1 et 4 autres selon la procédure 2. La variance entre les échantillons est significativement réduite avec la procédure 2.
- La Figure 5 représente la transparence des matrices de collagène obtenues selon les procédures 1 et 2, avec la condition #11 à 60 mg/ml.

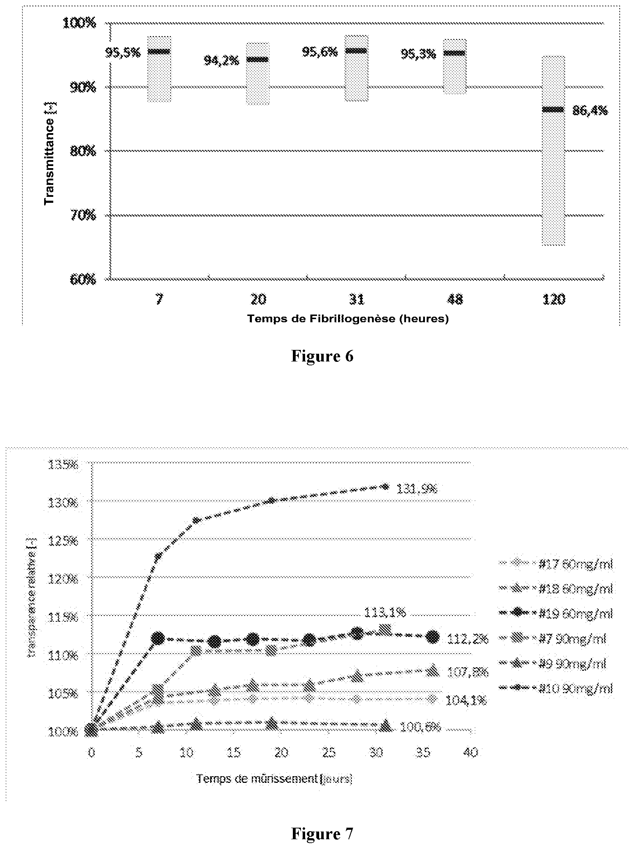

- La Figure 6 représente la Transparence des matrices de collagène avec la condition #11 à 60 mg/ml et à différentes durées d'exposition aux vapeurs d'ammoniac. Les bandes grisées représentent la transparence entre 380 nm et 780 nm, la moyenne de la transparence est indiquée par le trait noir.
- La Figure 7 représente des exemples de l'effet du temps de maturation sur les échantillons préparés dans différentes conditions physicochimiques et concentration de collagène. Le gain en transparence dépend fortement des deux paramètres. La transparence relative est représentée en ordonnée et le temps de maturation est représenté en abscisse.

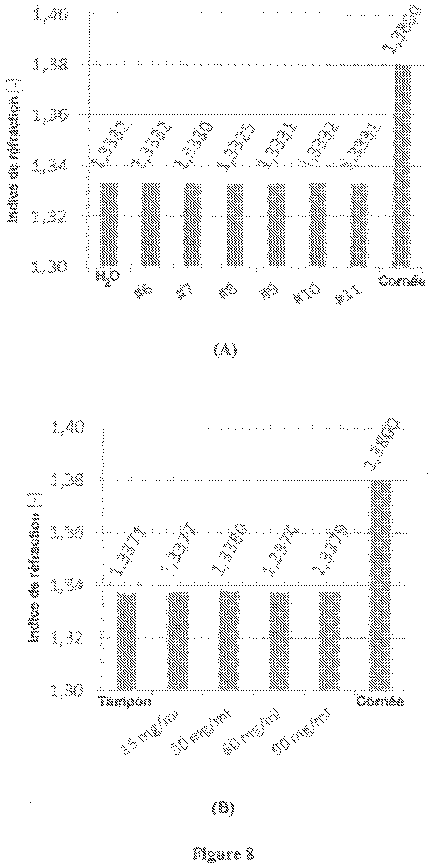

- La Figure 8 représente l'indice de réfraction de matrices de collagène à 584 nm ; A) des matrices de collagène à différentes conditions physicochimiques et à 60 mg/ml B) des matrices de collagène présentant un épithélium reconstitué *in vitro.*

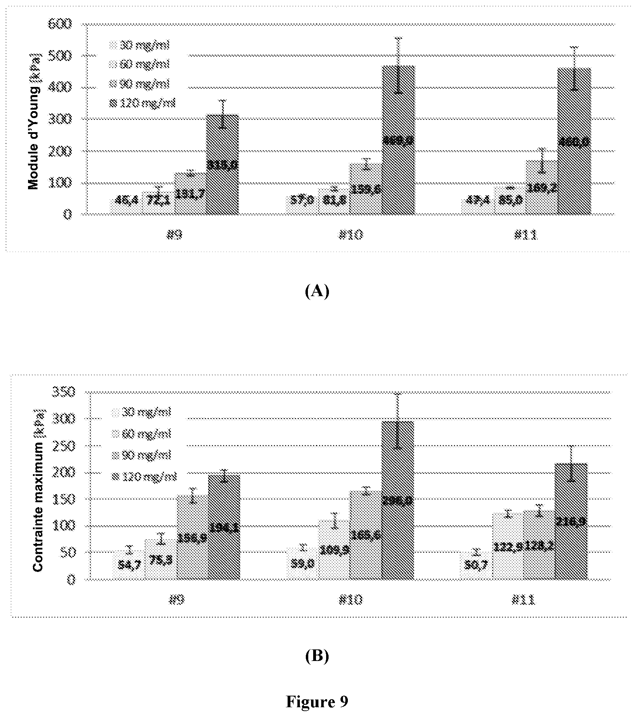

- Les Figures 9 (A)-(B) représentent la résistance mécanique des matrices fabriquées aux conditions #9, #10 et #11 et à différentes concentrations ; A) Module de Young, B) Contrainte de rupture. Le module de Young et la contrainte maximale augmentent avec la densité de collagène. Les barres d'erreurs indiquent l'erreur standard de la moyenne.

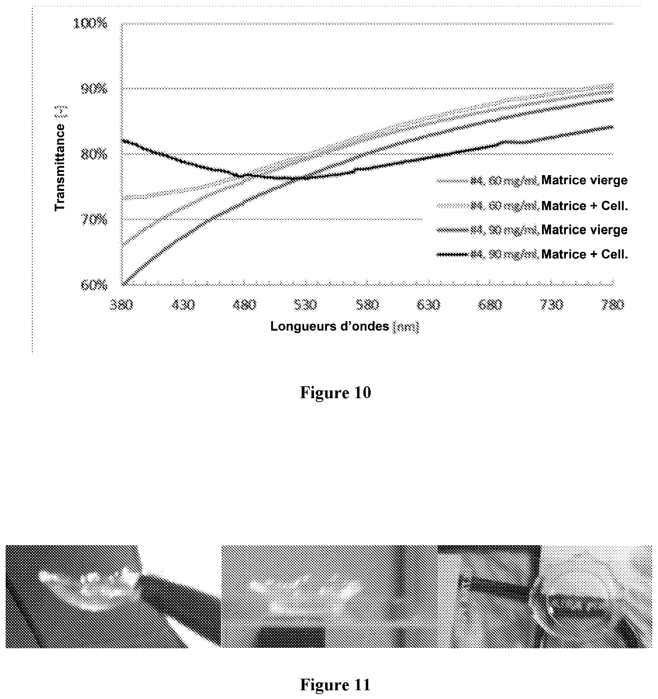

- La Figure 10 représente la transparence des matrices de collagène (condition #4, aux concentrations en collagène de 60 mg/ml et 90 mg/ml) sans et avec un épithélium reconstitué *in vitro.*
- La Figure 11 représente des photographies de matrices de collagène transparentes (condition #4, 60 mg/ml) obtenues avec le procédé de l'invention moulées de façon à mimer une cornée humaine.

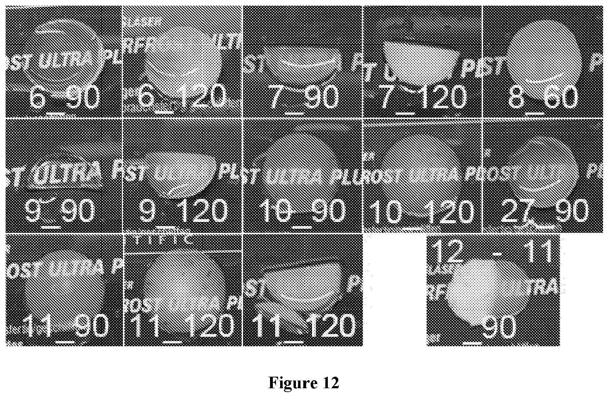

- La Figure 12 présente des photographies de différentes matrices moulées soit avec le procédé 1 soit avec le procédé 2 et suivant différentes concentrations et conditions physico-chimiques. Le texte sur lequel sont disposées les matrices est plus ou moins visible suivant la transparence de la matrice. La photographie 12-11_90 présente une matrice composite.

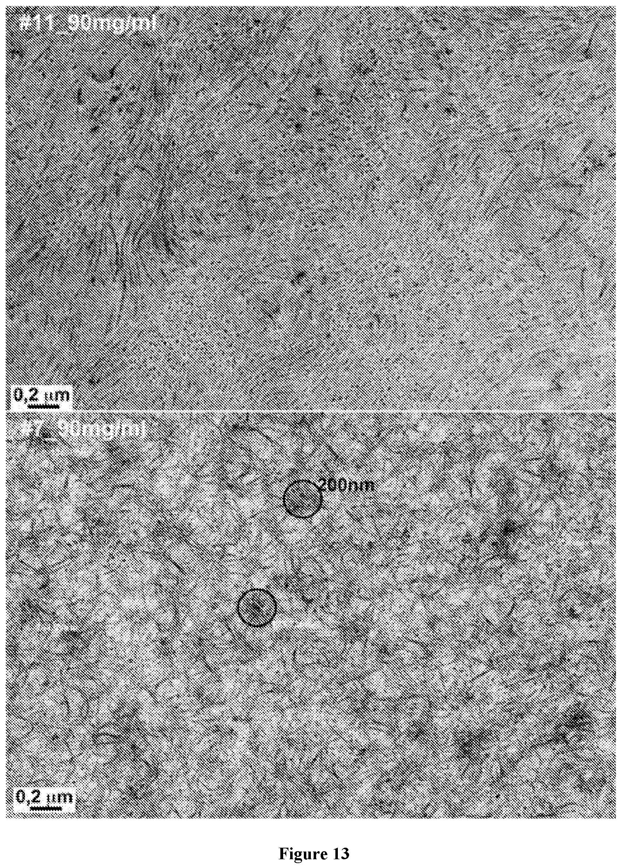

- La Figure 13 représente des images obtenues par Microscopie Électronique en Transmission de coupes ultrafines de matrices synthétisées à 90 mg/ml suivant les conditions #11 (haut) et #7 (bas). On constate la présence de fibrilles bien individualisée et relativement équidistantes et organisées en haut (#11) alors qu'en bas (#7), on observe des agrégats de fibrilles de 200 nm de diamètre espacés les uns des autres par des fibrilles fines réticulées et imposant une inter-distance relativement régulière.

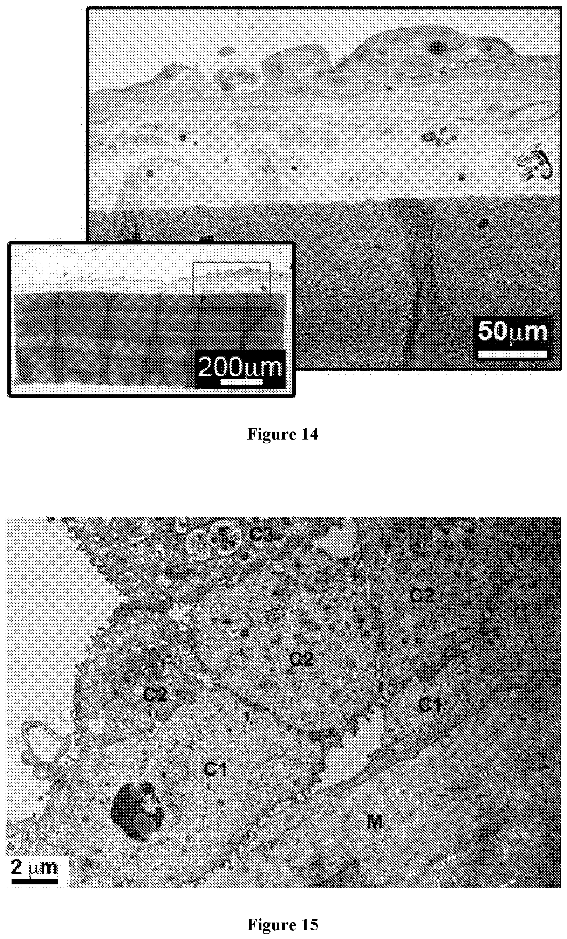

- La Figure 14 représente une coupe semi-fine d'une matrice de collagène (condition #11, 90 mg/ml) obtenues par microscopie optique. En bas à gauche une vue d'ensemble et à droite un zoom de la zone encadrée à gauche. On observe la matrice dense ainsi que la couche de cellules épithéliales s'étant développée à la surface.
- La Figure 15 représente une image obtenue par Microscopie Électronique en Transmission montrant la couche cellulaire sur une matrice de collagène (condition #4, 60 mg/ml). Les cellules sont adhérentes à la matrice et forment une couche épithéliale.

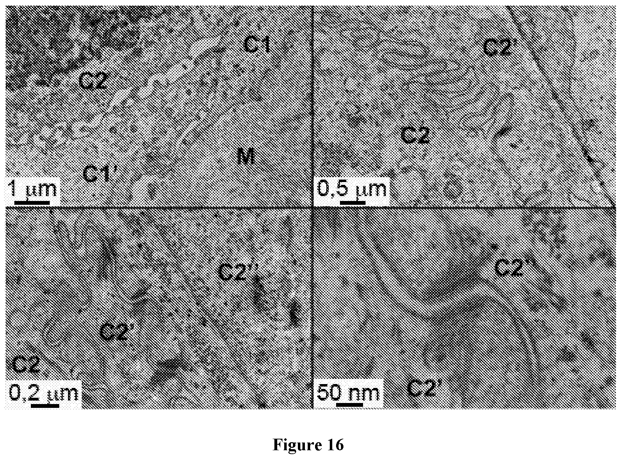

- La Figure 16 représente un montage d'images obtenues par Microscopie Électronique en Transmission de coupes ultrafines d'une matrice de collagène (condition #11, 90 mg/ml) montrant les ultrastructures typiques des cellules épithéliales : en haut à gauche les empilements cellulaires sur la matrice ; en haut à droite, les interdigitations entre cellules latérales ; en bas les desmosomes assurant l'adhésion des cellules entre elles et le passage d'information et de nutriments (à gauche une vue à faible grandissement, à droite un zoom sur le desmosome central).

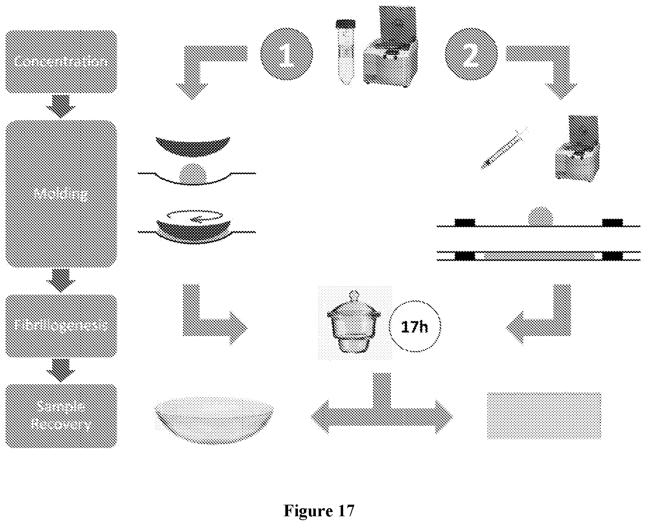

- La Figure 17 représente un schéma illustrant les procédés de synthèse des matrices de collagène 1 et 2 selon l'invention.

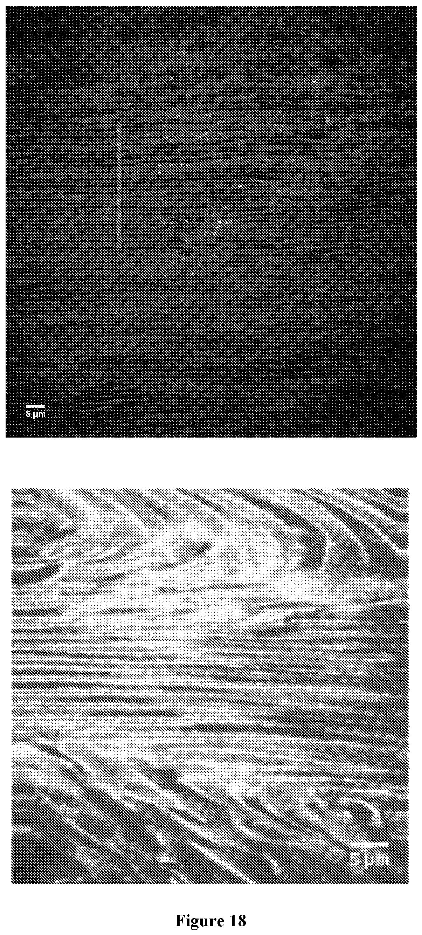

- La Figure 18 représente des images obtenues par Génération de Seconde Harmonique d'une solution de collagène (condition #36 : 0,3 mM HCl et 10 mM CH₃COOH) injectée en microcellule en verre. Ces images permettent de vérifier le type d'organisation cristal liquide obtenu à partir de la condition physico-chimique initiale. Le profil d'intensité de ces images est typique de celui décrit pour un nématique contre-plaqué.

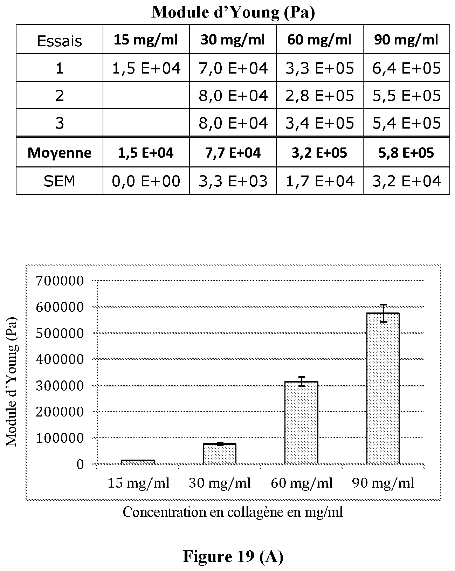

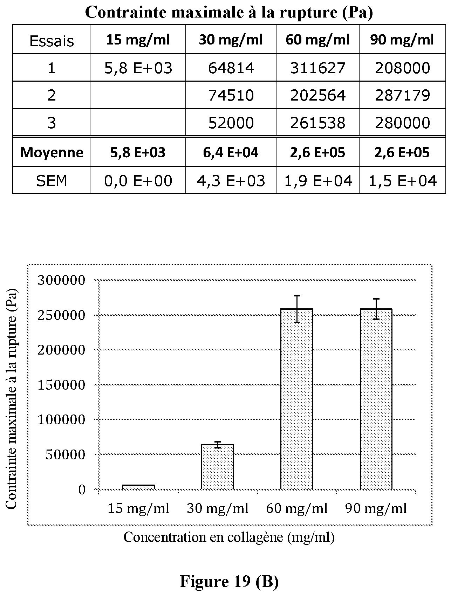

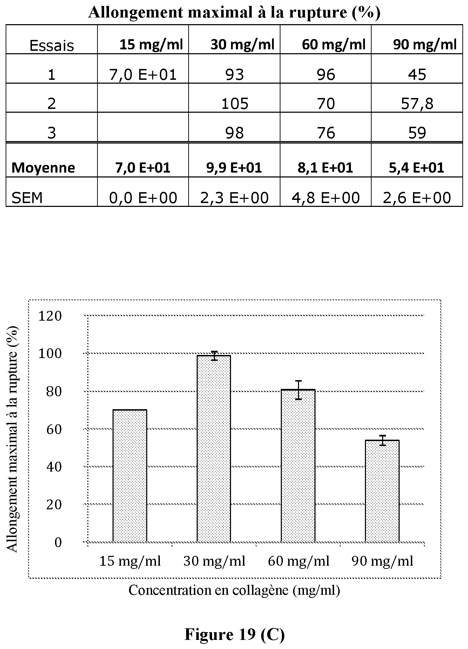

- Les Figures 19 (A)-(C) représentent la résistance mécanique des matrices de collagènes fabriquées aux conditions #4 et à différentes concentrations : (A) Module de Young exprimé en unité Pa ; (B) Contrainte maximale à la rupture exprimée en unité Pa ; (C) Allongement maximal à la rupture exprimé en % d'élongation par rapport à la longueur initiale. Le module de Young et la contrainte maximale augmentent avec la densité de collagène contrairement à l'élongation à la rupture qui, elle, diminue avec la concentration en collagène. Les barres d'erreurs indiquent l'erreur standard de la moyenne.
   Dans les Figures 19 (A)-(C), SEM désigne l'Erreur Standard de la Moyenne ou « Standard Error of the Mean » en anglais. Elle se calcule en divisant l'écart-type de l'échantillon (« standard deviation ») par la racine carrée du nombre d'échantillons testés (σ / √n).
- La Figure 20 représente la transparence des matrices de collagène fibrillé dans des conditions #35, #36 et #37 avec une concentration de collagène de 60 mg/ml.
- La Figure 21 représente la transparence des matrices de collagène fibrillé dans des conditions #36 avec une concentration de collagène de 90 mg/ml.

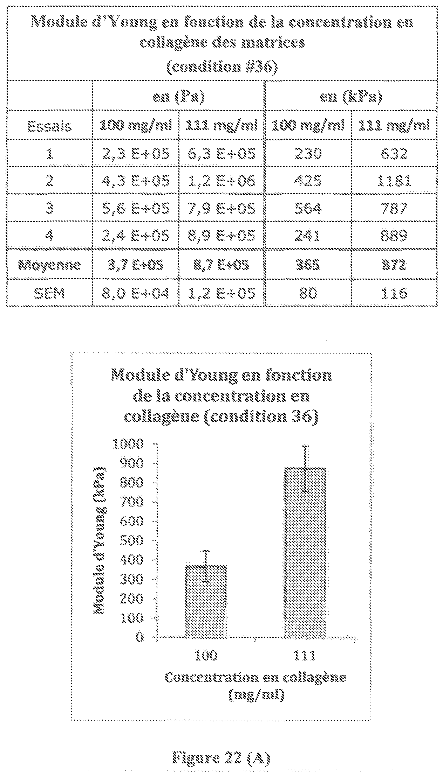

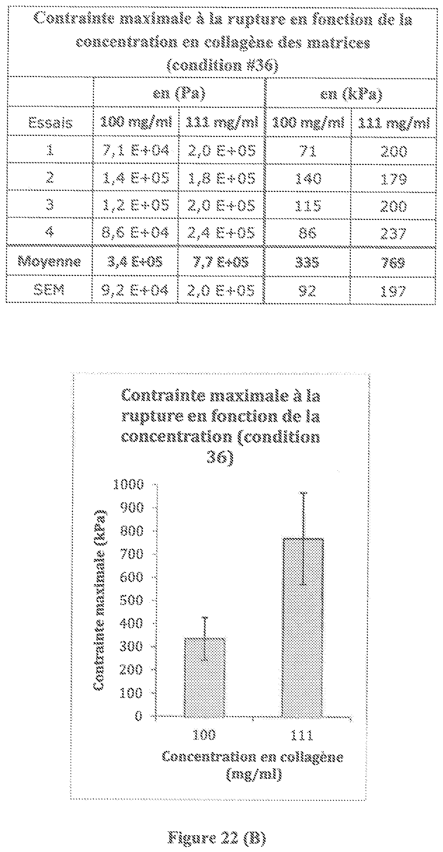

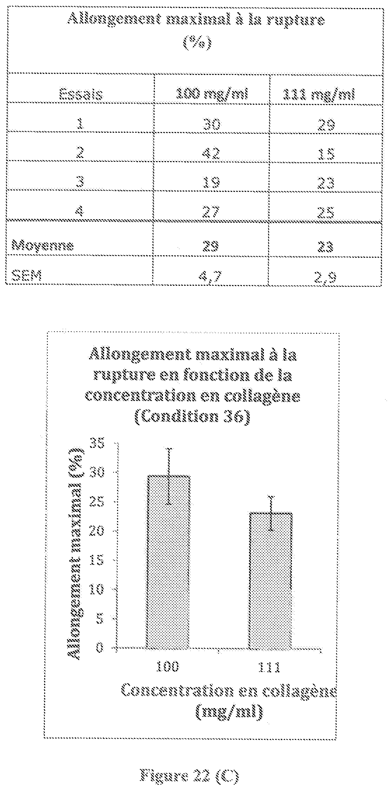

- Les Figures 22 (A)-(C) représentent la résistance mécanique des matrices de collagènes fabriquées aux conditions #36 et à différentes concentrations : (A) Module de Young exprimé en unité Pa et kPa ; (B) Contrainte maximale à la rupture exprimée en unité Pa et kPa ; (C) Allongement maximal à la rupture exprimé en % d'élongation par rapport à la longueur initiale. Le module de Young et la contrainte maximale augmentent avec la densité de collagène contrairement à l'élongation à la rupture qui elle diminue avec la concentration en collagène. Les barres d'erreurs indiquent l'erreur standard de la moyenne. SEM désigne l'Erreur Standard de la Moyenne ou « Standard Error of the Mean » en anglais. Elle se calcule en divisant l'écart-type de l'échantillon (« standard deviation ») par la racine carrée du nombre d'échantillons testés (σ / √n).
- La Figure 23 représente les diamètres des fibrilles de collagène dans les matrices synthétisées à différentes concentrations de collagènes (15 mg/ml, 30 mg/ml, 60 mg/ml et 90 mg/ml) préparées dans les conditions #4. Les fibrilles en coupe transverse ont été détectées par traitement d'images de microscopie électronique par le logiciel Image J. Les erreurs standard et intervalles de confiance ont été calculés par les fonctions automatiques du logiciel Microscoft Excel.

### EXEMPLES

La présente invention est décrite en détail à l'aide des exemples suivants, auxquels elle n'est cependant pas limitée.

Dans les exemples, un collagène de type I préparé à partir de queues de jeunes rats Wistar, selon le mode opératoire décrit par Gobeaux F et al. (Langmuir, 2007, 23, 641 1-6417) a été utilisé.

Les tendons de queue de rat sont excisés dans une hotte à flux laminaire stérile, puis lavés dans une solution tampon phosphate saline contenant 137 mM de NaCl, 2,68 mM de KCl, 8,07 mM de Na₂HPO₄, et 1,47 mM de KH₂PO₄, pour éliminer les cellules et les traces de sang. Les tendons sont ensuite trempés dans une solution de NaCl 4 M pour éliminer les cellules intactes restantes et précipiter une partie des protéines à poids moléculaire élevé. Après un nouveau lavage avec la solution tampon saline, et de l'eau miliQ, les tendons sont dissous dans une solution aqueuse à 500 mM d'acide acétique. La solution ainsi obtenue est clarifiée par centrifugation à 4 500 rpm (4922g) pendant 40 minutes à 10°C ; puis à 21 000 rpm (53343 g) pendant 2 heures à 10°C. Une première partie des protéines autres que le collagène de type I est précipitée de manière sélective dans une solution aqueuse de NaCl 300 mM, puis éliminée par centrifugation à 21 000 rpm (53343 g) pendant 2 heures à 10°C. Le collagène I est récupéré à partir du surnageant par précipitation dans une solution aqueuse de NaCl 700 mM suivie d'une centrifugation à 4500 tr/min ou rpm (4922g) pendant 1 heure à 10°C. Les culots ainsi obtenus sont dissous dans une solution aqueuse d'acide acétique 500 mM, puis dialysés plusieurs fois contre de l'acide acétique 500 mM pour éliminer totalement le NaCl. Les solutions sont maintenues à 4 °C puis centrifugées à 21 000 rpm (53343 g) pendant 3 heures à 10°C. La concentration en collagène de la solution acide a été déterminée par dosage d'hydroxyproline. Bien entendu, d'autres sources de collagène peuvent être utilisées. Une purification peut être alternativement effectuée en remplaçant l'acide acétique 500 mM par de l'acide chlorhydrique à 2,5 mM. La solution de collagène présente une concentration en collagène de 3,16 mg/ml et une concentration en acide acétique de 500 mM (acide chlorhydrique 2,5mM). Le pH de la solution de collagène est de 2,5. La solution est stockée dans des tubes stériles de 45 ml à 4°C. La solution de collagène ainsi obtenue est désignée comme solution initiale de collagène dans ce qui suit.

### EXEMPLE 1 : Préparation des solutions de collagène pour la synthèse des matrices

Les inventeurs ont mis en place un plan d'expérience afin de déterminer l'effet de l'acide acétique et de l'acide chlorhydrique et de leurs concentrations respectives sur la préparation de matrices de collagènes.

Des extraits de la solution initiale de collagène monomérique acido-soluble ont chacun été dialysés contre des solutions comprenant de l'acide chlorhydrique et/ou de l'acide acétique de façon à obtenir différentes solutions de collagène pour la synthèse de matrices. Les différentes solutions de collagène utilisées pour la synthèse de matrices sont décrites en Figure 1.

Ainsi, un certain nombre de solutions de collagène ont été préparées pour la synthèse de matrices. Chaque solution de collagène préparée présente une concentration définie en acide acétique et une concentration en acide chlorhydrique. Ces solutions de collagène pour la synthèse de matrices ont été concentrées par la technique de filtration par centrifugation.

### Dialyse des solutions initiales de collagène

La solution de collagène initiale est dialysée contre une solution comprenant un mélange d'acide acétique et d'acide chlorhydrique. La concentration en acide acétique et en acide chlorhydrique de chaque solution de collagène pour la synthèse de matrices peut être ajustée par des cycles répétés de dialyse. Une membrane de dialyse de 30 kDa est utilisée. La dialyse est effectuée pendant environ 12 heures. Une mesure de pH est réalisée afin de confirmer que le cycle de dialyse a permis d'obtenir la solution désirée.

### Concentration des solutions de collagène dialysées par la technique de filtration par centrifugation

Les solutions dialysées sont placées dans des concentrateurs de 20 ml (Vivaspin 30kD de cutoff) avant d'être centrifugées à basse vitesse sur une centrifugeuse Beckman-Coulter J26-XP (4500 tr/min. - 4922 g, 10°C) à angle variable. En fin du processus de concentration, le milieu réceptionné dans la partie basse du tube est éliminé pour éviter une réhydratation de la solution concentrée.

Toutes les différentes solutions ont ainsi été concentrées jusqu'à 60 mg/ml en collagène et certaines, notamment celles des conditions #9, #10, #11 et #36 ont aussi été concentrées jusqu'à 90, 100, 110 et 120 mg/ml en collagène. Les solutions de collagènes ainsi obtenues sont stockées à 4°C.

L'étape de concentration permet d'avoir, avant la fibrillogenèse, un collagène sous forme cristal liquide avec une organisation ordonnée et hiérarchique des molécules de collagène. Par exemple, il a été observé qu'à l'issue de l'étape de concentration, les solutions concentrées (60-80 mg/ml ≤) des conditions #4, #9, #10 et #11 ont une organisation cristal liquide du type cholestérique alors que celles des conditions #34, #35, #36, #37 et #38 (45-80 mg/ml ≤) ont une organisation en nématique contreplaqué. Le type d'organisation cristal liquide obtenu pour les différents échantillons de collagène avant la fibrillogenèse peut être déterminé, notamment par microscopie photonique entre polariseurs croisés et par la technique de microscopie optique par génération de second harmonique.

La Figure 18 montre bien une organisation en contreplaqué d'une matrice de collagène (condition #36).

### EXEMPLE 2 : Préparation des matrices de collagène fibrille

### Moulage des solutions de collagène concentrées

Les solutions de collagène concentrées sont placées soit dans un moule céramique incurvé et pressé avec une contrepartie plastique (procédé 1), soit dans un moule constitué de deux plaques de verres planes maintenues écartées par des entretoises d'épaisseur définies (procédé 2) (Figure 17). Le procédé 1 montre la possibilité d'obtenir une matrice mimant la forme d'une cornée (Figure 11).

### Fibrillogenèse de solutions de collagène concentrées

La fibrillogenèse est induite par l'exposition des solutions concentrées de collagène (éventuellement disposées dans un moule) sous vapeur saturante d'ammoniac (37%) dans une enceinte close (3 1) à 20°C pendant 7 à 48 heures. Une matrice de collagène fibrillé est obtenue. Les matrices de collagènes peuvent éventuellement être démoulées.

### Mûrissement des matrices de collagène

Éventuellement, les matrices de collagène démoulées sont immergées dans de l'eau déminéralisée. Les matrices sont alors laissées dans l'eau à 20°C ou 4°C pendant un mois. Cette étape peut favoriser la croissance longitudinale des fibrilles de collagène et la stabilisation des matrices de collagène. Les matrices peuvent ensuite être stockées à 4°C avant utilisation (culture cellulaire ou implantation *in vivo*).

### EXEMPLE 3 : Transparence des matrices de collagène fibrillé

### La transparence est fonction de l'état physico-chimique au cours de la fibrillogenèse

Différentes conditions physico-chimiques différentes ont été évaluées afin d'optimiser les paramètres pour la synthèse d'une matrice transparente de collagène. Dans un premier temps, les matrices ont été fabriquées avec une concentration de collagène de 60 mg/ml, selon la procédure 1. Dans les conditions #9, #10 et #11 des matrices de collagène ont été obtenues avec des concentrations en collagène de 90 mg/ml et de 120 mg/ml. Des matrices de collagène ont été également obtenues avec une concentration en collagène de 90 mg/ml dans les conditions #36. Les résultats montrent qu'on obtient des matrices de collagène transparentes pour une partie des conditions testées (Figure 2A). Lorsque l'on considère l'inverse du coefficient d'absorption (1/αc), on remarque qu'un groupe d'échantillon caractérisé par des concentrations en acide acétique relativement faibles se détache (Figure 2B). Les conditions #9 - #11, #34 - #38 permettent d'obtenir la transparence la plus élevée et ont donc été étudiées plus particulièrement.

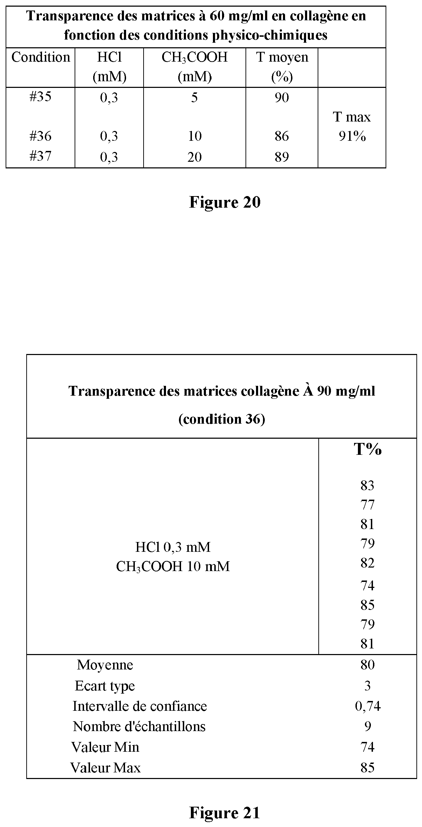

Les Figures 2, 3, 20 et 21 montrent les résultats de transparence d'une matrice de collagène obtenue dans ces conditions.

### Effet de la concentration de collagène sur la transparence des matrices de collagène.

D'une façon générale, plus la concentration en collagène est élevée, plus la transparence de la matrice obtenue est faible. Cependant, il semble que la diminution de transparence dépende des conditions physico-chimiques spécifiques au cours de la fibrillogenèse. Cet effet est illustré par la Figure 3. Pour les trois conditions #9, #10 et #11, une série supplémentaire d'échantillons a été fabriqué à différentes concentrations de collagènes, allant de 90 mg/ml à 120 mg/ml par paliers de 10 mg/ml (voir Figure 3B).

Les matrices de collagène obtenues selon la procédure 2 proviennent de la même série (entretoises d'approximativement 500 µm). Les matrices de collagène sont donc supposées présenter la même épaisseur. Par conséquent, la densité optique de la matrice n'a pas été ajustée à l'épaisseur de l'échantillon afin d'éviter l'incertitude d'une mesure supplémentaire. La longueur d'onde a été limitée à la gamme de 530 nm à 580 nm pour des raisons de commodités expérimentales.

Quel que soit le protocole, la tendance générale de diminution de la transparence des matrices de collagène avec l'augmentation de la concentration en collagène a pu être reproduite expérimentalement, notamment avec la condition #9.

### Indice de réfraction des matrices de collagène

Les indices de réfraction à 580 nm de matrices de collagène nues ou présentant une couche épithéliale développée à partir d'explants limbiques humains ont été mesurés. Les matrices de collagène sans cellules présentent un indice réfraction de 1,333 (Figure 8A). Les matrices de collagène avec des cellules épithéliales présentent un indice de réfraction de 1,337 (Figure 8B). Ceci suggère que les matrices de collagène synthétisées dans une condition donnant lieu à un cholestérique et en absence de l'étape de relaxation préalable à l'étape de fibrillogenèse, présentant ou non un épithélium, n'induisent pas une réfraction significative de la lumière (Figure 8).

### EXEMPLE 4 : Reproductibilité des matrices de collagène et variance des résultats

Les modifications qui ont été apportées au protocole de fabrication ont pour but d'augmenter la reproductibilité des mesures afin de pouvoir mieux évaluer les meilleures conditions de synthèse. L'utilisation d'un moule plan avec entretoise permet de réduire la variance entre les échantillons de mêmes conditions physico-chimiques. Ceci est illustré par la transparence des matrices de collagène pour la condition #11 à 60 mg/ml (Figure 4). Quatre échantillons ont été préparés pour chacune des deux différentes procédures de fabrication. Ces deux séries ont donné une transparence moyenne d'environ 96%, mais une diminution considérable de l'intervalle de confiance 2σ pourrait être obtenue avec la procédure 2 (diminution relative de 83%, voir Figure 5). Ceci suggère que la procédure 2 permet une reproductibilité satisfaisante des résultats, ce qui permet la quantification des propriétés macroscopiques tels que la transparence des matrices en effectuant des mesures sur trois ou cinq échantillons du même type.

### EXEMPLE 5 : Effet de l'exposition prolongée aux vapeurs d'ammoniac sur la transparence des matrices de collagène

Les échantillons sont laissés 17 heures sous vapeur d'ammoniac pour induire la fibrillogenèse. Un ensemble de matrices de collagènes obtenues en condition #11 à 60 mg/ml a été exposé à la vapeur d'ammoniac pendant des temps différents. On observe que lorsque les matrices de collagène subissent une exposition prolongée de l'ordre de 120 heures, leur transparence diminue significativement (Figure 6). Aucune modification notable n'est par contre observée jusqu'à 48 heures d'imprégnation.

### EXEMPLE 6 : Maturation des matrices au cours du temps

Pour tous les échantillons une évolution de la transparence a été observée au cours du temps. En général, la transparence augmente au cours des deux à trois premières semaines avant d'atteindre une valeur seuil. Le gain spécifique de transparence dépend de la condition physico-chimique au cours de la fibrillogenèse (Figure 7). Pour certaines conditions, le gain est de l'ordre de 1%, pour d'autres il a atteint des niveaux allant jusqu'à 32%.

### EXEMPLE 7 : Propriétés mécaniques des matrices de collagène

Il existe une relation directe entre les propriétés mécaniques des matrices de collagène et leur densité en collagène.

Cette relation a été observée pour la rigidité, représentée par le module de Young (Figure 9A), ainsi que pour la contrainte mécanique maximale supportée par les matrices de collagène, représentée par la contrainte maximale à la rupture (Figure 9B).

Cette relation directe entre les propriétés mécaniques des matrices de collagène et leur densité en collagène a, en outre, été observée pour la rigidité de matrices de collagène à différentes concentrations de collagènes (15 mg/ml, 30 mg/ml, 60 mg/ml et 90 mg/ml) préparées dans les conditions #4. Les Figures 19(A)-(C) représentent pour ces matrices de collagène, le module de Young ou module d'élasticité (longitudinale) exprimé en unité Pa ; la contrainte mécanique maximale supportée par ces matrices représentée par la contrainte de rupture exprimée en unité Pa ; et la capacité de ces matrices à s'allonger avant de rompre lorsqu'elles sont sollicitées en traction exprimée en %.

La relation directe entre les propriétés mécaniques des matrices de collagène et leur densité en collagène a, également, été observée pour la rigidité de matrices de collagène à différentes concentrations de collagènes (100 mg/ml et 111 mg/ml) préparées dans les conditions #36. Les Figures 22 (A)-(C) représentent pour ces matrices de collagène, le module de Young ou module d'élasticité (longitudinale) exprimé en unité Pa et kPa ; la contrainte mécanique maximale supportée par ces matrices représentée par la contrainte de rupture exprimée en unité Pa et kPa ; et la capacité de ces matrices à s'allonger avant de rompre lorsqu'elles sont sollicitées en traction exprimée en %.

### EXEMPLE 8 : Diamètre des fibrilles de collagène dans les matrices synthétisées

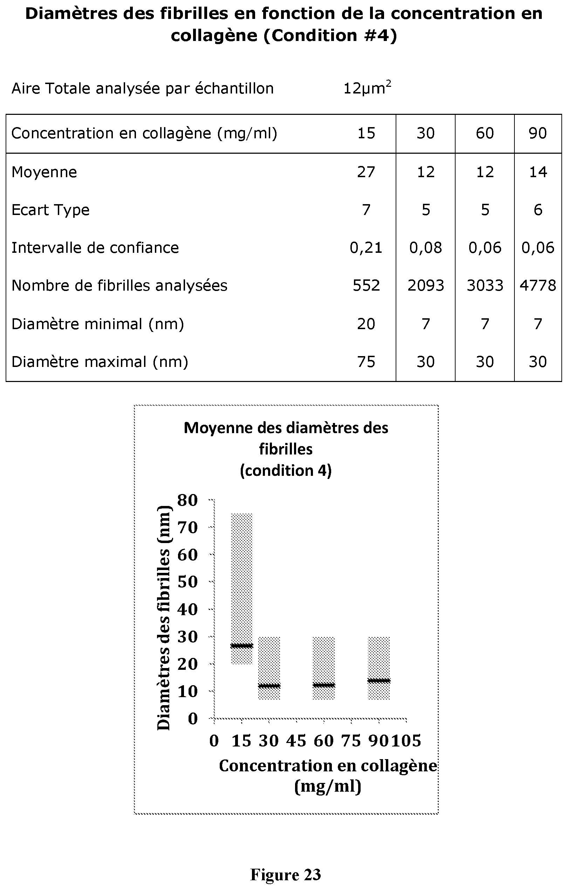

Les diamètres des fibrilles de collagène dans les matrices synthétisées à différentes concentrations de collagènes (15 mg/ml, 30 mg/ml, 60 mg/ml et 90 mg/ml) préparées dans les conditions #4 ont été mesurés (Figure 23). Les fibrilles en coupe transverse ont été détectées automatiquement par traitement d'images de microscopie électronique par le logiciel Image J. Ce traitement nécessite de fixer un seuil arbitraire, un intervalle d'aire ainsi qu'un facteur d'élongation. Un masque est alors généré avec toutes les densités sélectionnées automatiquement par le programme. Ce masque est comparé avec l'image de départ afin de vérifier que les densités sélectionnées automatiquement correspondent bien globalement à des coupes transverse de fibrille. Les erreurs standard et intervalles de confiance ont été calculés en utilisant les fonctions automatiques du logiciel Microscoft Excel (2008). Le diamètre moyen des fibrilles à 15 mg/ml est plus grand que celui des fibrilles aux concentrations plus élevées. Il a été constaté que malgré l'augmentation de la concentration en collagène de 30 à 90 mg/ml, le diamètre des fibrilles reste comparable.

### EXEMPLE 9 : Mise en culture d'explants limbiques sur les matrices de collagène et effets sur leur transparence

Pour estimer le développement de cellules épithéliales sur les matrices de collagène, des matrices de collagène de la condition #4 (condition 60 mg/ml, 90 mg/ml) ont été lavées avec de l'eau pure (Milli Q) puis avec du milieu de culture. Des explants limbiques humains ont été cousus aux matrices de collagène afin de permettre la migration cellulaire. De telles matrices ont été immergées dans un milieu de culture pendant 14 jours. Les cultures ont été ensuite continuées pendant 10 jours, la face antérieure des matrices de collagène étant laissée à l'air libre, permettant ainsi la génération d'un épithélium. Enfin, les matrices de collagène ont été utilisées pour des observations ultérieures.

L'observation microscopique des cultures cellulaires permet de montrer que les cellules épithéliales sont viables et prolifèrent sur les matrices de collagène. Les cellules migrent depuis l'explant naturel, et s'étendent pour coloniser la surface des matrices de collagène (Figures 14 et 15). Les ultra-structures caractéristiques des cellules épithéliales sont bien observées par microscopie électronique en transmission (Figure 16).

La transparence des matrices de collagène a été mesurée avant le début de la culture cellulaire puis au bout de 24 jours. Les résultats montrent que la transparence de la matrice est peu affectée par la colonisation cellulaire (Figure 10).

### EXEMPLE 10 : Synthèse de matrices composites

Des solutions de conditions physico-chimiques différentes peuvent être agencées les unes à coté des autres pour former des matrices composites (Figure 12).

## Revendications

1. Procédé de préparation d'une matrice de collagène fibrillé **caractérisé en ce qu'**il comprend les étapes suivantes :
(a) la préparation d'une solution aqueuse acide comprenant
- du collagène sous forme de monomères à une concentration comprise entre 0,1 et 10 mg/ml, avantageusement comprise entre 0,3 et 5 mg/ml, et
- au moins un acide fort présent en une concentration de 0,01 à 10 mM et un acide faible présent en une concentration comprise entre 0,1 et 500 mM,
(b) la concentration de la solution obtenue en (a) jusqu'à une concentration en collagène comprise entre 10 et 250 mg/ml ;
(c) la formation des fibrilles par traitement de la solution de collagène obtenue à l'étape (b) par une phase gazeuse basique ou par une phase liquide basique.

2. Procédé selon la revendication 1, **caractérisé en ce que** l'acide faible est choisi parmi l'acide acétique, l'acide chloro-, dichloro-, trichloro- acétique; l'acide bromoacétique ; l'acide trifluoro-acétique ; l'acide propionique ; l'acide ascorbique ; l'acide valérique ; l'acide 2-, 4-, 5- bromovalérique ; l'acide lactique ; l'acide citrique ; l'acide salicylique ; l'acide acétylsalicylique ; l'acide formique, les acides de la famille des acides uroniques, de préférence l'acide glucuronique ; l'acide galacturonique ;
et
- l'acide fort est choisi parmi l'acide chlorhydrique ; l'acide sulfurique ; l'acide perchlorique ; l'acide fluorhydrique ; l'acide iodhydrique ; l'acide chlorique ; l'acide permanganique ; l'acide manganique.

3. Procédé selon l'une des revendications 1 ou 2, **caractérisé en ce que** :
- l'acide faible est présent en une concentration comprise entre 0,2 à 200 mM, et préférentiellement de 2 à 125 mM ; et
- l'acide fort est présent en une concentration de 0,1 à 3 mM et préférentiellement de 0,2 à 2,5 mM.

4. Procédé selon l'une quelconque des revendications 1 à 3, **caractérisé en ce que** l'acide fort est l'acide chlorhydrique et l'acide faible est l'acide acétique.

5. Procédé selon l'une quelconque des revendications 1 à 4, **caractérisé en ce qu'**à l'étape (b), la concentration de la solution est réalisée par la filtration par centrifugation, par l'ultrafiltration, par évaporation rapide sous cryo-centrifugation ou par l'osmose inverse, avantageusement par la filtration-centrifugation.

6. Procédé selon l'une quelconque des revendications 1 à 5, **caractérisé en ce que** la concentration en collagène à l'issue de l'étape b) est comprise entre 10 et 250 mg/ml, préférentiellement entre 30 et 200 mg/ml, et avantageusement entre 40 et 120 mg/ml.

7. Procédé selon l'une quelconque des revendications 1 à 6, **caractérisé en ce qu'**il comporte, en outre, après l'étape de concentration (b) et avant l'étape de formation des fibrilles (c), une étape de mise en forme de la solution de collagène et/ou une étape de relaxation.

8. Procédé selon la revendication 7, **caractérisé en ce que** l'étape de mise en forme est une étape de moulage.

9. Procédé selon l'une quelconque des revendications 1 à 8, **caractérisé en ce qu'**à l'étape (c), le traitement par une phase gazeuse basique est un traitement par la vapeur d'ammoniac.

10. Procédé selon l'une quelconque des revendications 1 à 9, **caractérisé en ce qu'**il comporte, en outre, après l'étape de formation des fibrilles (c), une étape de mûrissement.

11. Procédé de préparation d'une matrice de collagène fibrillé transparente selon l'une quelconque des revendications 1 à 10, comprenant les étapes suivantes :
(a) la préparation d'une solution acide aqueuse comprenant :
- du collagène, avantageusement de type I, éventuellement associé à d'autres types de collagènes, à une concentration comprise entre 0,1 et 10 mg/ml, avantageusement comprise entre 0,3 et 5 mg/ml, et
- au moins de l'acide acétique et de l'acide chlorhydrique.
(b) la concentration de la solution obtenue en (a) par filtration centrifugation jusqu'à une concentration en collagène comprise entre 10 et 250 mg/ml, préférentiellement entre 30 et 200 mg/ml, et avantageusement entre 40 et 120 mg/ml ;
(c) la mise en forme de la solution de collagène obtenue en (b) ;
(d) la relaxation de la solution de collagène à l'issue de l'étape (c) ;
(e) la formation des fibrilles par traitement de la solution de collagène obtenue à l'étape (d) par une phase gazeuse basique ;
(f) le mûrissement des fibrilles obtenues à l'étape (e) jusqu'à obtention d'une matrice.

12. Matrice de collagène fibrillé transparente susceptible d'être obtenue par le procédé selon l'une quelconque des revendications 1 à 11, **caractérisée en ce qu'**elle transmet au moins 80% de la lumière blanche.

13. Matrice de collagène fibrillé transparente selon la revendication 12, **caractérisée en ce que** sa densité en collagène est égale ou supérieure à 40 mg/ml.

14. Matrice de collagène fibrillé composite susceptible d'être obtenue par le procédé selon l'une quelconque des revendications 1 à 10, **caractérisé en ce qu'**elle comporte au moins une zone opaque qui transmet moins de 80% de la lumière blanche et au moins une zone transparente qui transmet au moins 80% de la lumière blanche.

15. Utilisation d'une matrice transparente ou composite selon l'une quelconque des revendications 12 à 14 pour la fabrication d'un tissu ou d'un organe artificiel, notamment d'un substitut de cornée ou d'un substitut de cornée-sclère.
**Figure 1**
| **Condition** | **c(HCl) [mmol/l]** | **c(CH₃COOH) [mmol/l]** | **pH Mesuré** |
|---|---|---|---|
| **#4** | 1,25 | 70 | |
| **#6** | 0,30 | 120 | 2,86 |
| **#7** | 0,30 | 220 | 2,61 |
| **#8** | 0,30 | 320 | 2,57 |
| **#9** | 0,60 | 36 | 2,91 |
| **#10** | 0,90 | 36 | 2,85 |
| **#11** | 1,25 | 36 | 2,78 |
| **#12** | 0,90 | 120 | 2,69 |
| **#13** | 0,90 | 220 | 2,59 |
| **#14** | 0,60 | 220 | 2,60 |
| **#15** | 0,60 | 120 | 2,73 |
| **#20** | 1,25 | 200 | 2,67 |
| **#21** | 1,25 | 300 | 2,64 |
| **#22** | 1,25 | 500 | 2,53 |
| **#24** | 1,25 | 800 | 2,33 |
| **#24** | 1,50 | 36 | 2,53 |
| **#27** | 1,50 | 120 | 2,69 |
| **#30** | 1,50 | 220 | 1,50 |
| **#33** | 0,30 | 36 | 0,30 |
| **#34** | 1,25 | 120 | 1,25 |
| **#35** | 0,3 | 5 | |
| **#36** | 0,3 | 10 | |
| **#37** | 0,3 | 20 | |
| **#38** | 0,3 | 40 | |

## Patentansprüche

1. Verfahren zur Herstellung einer fibrillierten Kollagenmatrix, **dadurch gekennzeichnet, dass** es die folgenden Schritte umfasst:
(a) das Herstellen einer sauren wässrigen Lösung, umfassend
- Kollagen in Form von Monomeren mit einer Konzentration im Bereich zwischen 0,1 und 10 mg/ml, vorteilhafterweise im Bereich zwischen 0,3 und 5 mg/ml, und
- mindestens eine starke Säure, die in einer Konzentration von 0,01 bis 10 mM vorliegt, und eine schwache Säure, die in einer Konzentration im Bereich zwischen 0,1 und 500 mM vorliegt,
(b) das Konzentrieren der in (a) erhaltenen Lösung bis auf eine Kollagenkonzentration im Bereich zwischen 10 und 250 mg/ml;
(c) das Bilden der Fibrillen durch Behandeln der im Schritt (b) erhaltenen Kollagenlösung mit einer basischen gasförmigen Phase oder mit einer basischen flüssigen Phase.

2. Verfahren nach Anspruch 1, **dadurch gekennzeichnet, dass** die schwache Säure ausgewählt ist aus Essigsäure, Chlor-, Dichlor-, Trichloressigsäure; Bromessigsäure; Trifluoressigsäure; Propionsäure; Ascorbinsäure; Valeriansäure; 2-, 4-, 5-Bromvaleriansäure; Milchsäure; Zitronensäure; Salicylsäure; Acetylsalicylsäure; Ameisensäure, den Säuren aus der Familie der Uronsäuren, vorzugsweise Glucuronsäure; Galacturonsäure;
und
- die starke Säure ausgewählt ist aus Chlorwasserstoffsäure; Schwefelsäure; Perchlorsäure; Fluorwasserstoffsäure; Jodwasserstoffsäure; Chlorsäure; Permangansäure; Mangansäure.

3. Verfahren nach einem der Ansprüche 1 oder 2, **dadurch gekennzeichnet, dass**:
- die schwache Säure in einer Konzentration im Bereich zwischen 0,2 bis 200 mM, und vorzugsweise 2 bis 125 mM vorliegt; und
- die starke Säure in einer Konzentration von 0,1 bis 3 mM, und vorzugsweise 0,2 bis 2,5 mM vorliegt.

4. Verfahren nach einem der Ansprüche 1 bis 3, **dadurch gekennzeichnet, dass** die starke Säure Chlorwasserstoffsäure ist, und die schwache Säure Essigsäure ist.

5. Verfahren nach einem der Ansprüche 1 bis 4, **dadurch gekennzeichnet, dass** im Schritt (b) das Konzentrieren der Lösung durch Filtration durch Zentrifugation, durch Ultrafiltration, durch schnelles Verdampfen unter Kryozentrifugation oder durch Umkehrosmose, vorteilhafterweise durch Zentrifugationsfiltration ausgeführt wird.

6. Verfahren nach einem der Ansprüche 1 bis 5, **dadurch gekennzeichnet, dass** die Kollagenkonzentration am Ende des Schritts b) im Bereich zwischen 10 und 250 mg/ml, vorzugsweise zwischen 30 und 200 mg/ml, und vorteilhafterweise zwischen 40 und 120 mg/ml liegt.

7. Verfahren nach einem der Ansprüche 1 bis 6, **dadurch gekennzeichnet, dass** es weiter nach dem Schritt des Konzentrierens (b) und vor dem Schritt des Bildens der Fibrillen (c) einen Schritt zur Formgebung der Kollagenlösung und/oder einen Entspannungsschritt umfasst.

8. Verfahren nach Anspruch 7, **dadurch gekennzeichnet, dass** der Formgebungsschritt ein Abformschritt ist.

9. Verfahren nach einem der Ansprüche 1 bis 8, **dadurch gekennzeichnet, dass** im Schritt (c) das Behandeln mit einer basischen gasförmigen Phase ein Behandeln mit Ammoniakdampf ist.

10. Verfahren nach einem der Ansprüche 1 bis 9, **dadurch gekennzeichnet, dass** es weiter nach dem Schritt des Bildens der Fibrillen (c) einen Reifungsschritt beinhaltet.

11. Verfahren zur Herstellung einer transparenten fibrillierten Kollagenmatrix nach einem der Ansprüche 1 bis 10, das die folgenden Schritte umfasst:
(a) das Herstellen einer sauren wässrigen Lösung, umfassend:
- Kollagen, vorteilhafterweise vom Typ I, gegebenenfalls mit anderen Kollagentypen assoziiert, mit einer Konzentration im Bereich zwischen 0,1 und 10 mg/ml, vorteilhafterweise im Bereich zwischen 0,3 und 5 mg/ml, und
- mindestens Essigsäure und Chlorwasserstoffsäure.
(b) das Konzentrieren der in (a) erhaltenen Lösung durch Zentrifugationsfiltration bis auf eine Kollagenkonzentration im Bereich zwischen 10 und 250 mg/ml, vorzugsweise zwischen 30 und 200 mg/ml, und vorteilhafterweise zwischen 40 und 120 mg/ml;
(c) die Formgebung der in (b) erhaltenen Kollagenlösung;
(d) das Entspannen der Kollagenlösung am Ende des Schritts (c);
(e) das Bilden der Fibrillen durch Behandeln der im Schritt (d) erhaltenen Kollagenlösung mit einer basischen gasförmigen Phase;
(f) das Reifen der im Schritt (e) erhaltenen Fibrillen bis zum Erhalt einer Matrix.

12. Transparente fibrillierte Kollagenmatrix, die durch das Verfahren nach einem der Ansprüche 1 bis 11 erhalten werden kann, **dadurch gekennzeichnet, dass** sie mindestens 80 % des weißen Lichts durchlässt.

13. Transparente fibrillierte Kollagenmatrix nach Anspruch 12, **dadurch gekennzeichnet, dass** ihre Kollagendichte größer als oder gleich 40 mg/ml ist.

14. Zusammengesetzte fibrillierte Kollagenmatrix, die durch das Verfahren nach einem der Ansprüche 1 bis 10 erhalten werden kann, **dadurch gekennzeichnet, dass** sie mindestens eine opake Zone beinhaltet, die weniger als 80 % des weißen Lichts durchlässt, und mindestens eine transparente Zone, die mindestens 80 % des weißen Lichts durchlässt.

15. Verwendung einer transparenten oder zusammengesetzten Matrix nach einem der Ansprüche 12 bis 14 zur Produktion eines Gewebes oder eines künstlichen Organs, vor allem eines Hornhautersatzes oder eines Hornhaut-/Skleraersatzes.

## Claims

1. Method for preparing a fibrillated collagen matrix, **characterised in that** it comprises the following steps:
(a) preparing an acid aqueous solution comprising
- collagen in the form of monomers at a concentration comprised between 0.1 and 10 mg/ml, advantageously comprised between 0.3 and 5 mg/ml, and
- at least one strong acid present at a concentration of 0.01 to 10 mM and a weak acid present at a concentration comprised between 0.1 and 500 mM,
(b) the concentration of the solution obtained in (a) up to a collagen concentration comprised between 10 and 250 mg/ml;
(c) the formation of fibrils by treatment of the collagen solution obtained in step (b) by a basic gaseous phase or by a basic liquid phase.

2. Method according to claim 1, **characterised in that** the weak acid is selected from acetic acid, chloro-, dichloro-, trichloroacetic acid; bromoacetic acid; trifluoroacetic acid; propionic acid; ascorbic acid; valeric acid; 2-, 4-, 5-bromovaleric acid; lactic acid; citric acid; salicylic acid; acetylsalicylic acid; formic acid; acids from the uronic acid family, preferably glucuronic acid; galacturonic acid;
and
- the strong acid is selected from hydrochloric acid; sulphuric acid; perchloric acid; hydrofluoric acid; hydroiodic acid; chloric acid; permanganic acid; manganic acid.

3. Method according to one of claims 1 or 2, **characterised in that**:
- the weak acid is present at a concentration comprised between 0.2 and 200 mM, and preferably from 2 to 125 mM; and
- the strong acid is present at a concentration from 0.1 to 3 mM and preferably from 0.2 to 2.5 mM.

4. Method according to any one of claims 1 to 3, **characterised in that** the strong acid is hydrochloric acid and the weak acid is acetic acid.

5. Method according to any one of claims 1 to 4, **characterised in that** in step (b), the concentration of the solution is achieved by filtering by centrifugation, by ultrafiltration, by rapid evaporation under cryo-centrifugation or by reverse osmosis, advantageously by filtration-centrifugation.

6. Method according to any one of claims 1 to 5, **characterised in that** the collagen concentration at the end of step (b) is comprised between 10 and 250 mg/ml, preferably between 30 and 200 mg/ml, and advantageously between 40 and 120 mg/ml.

7. Method according to any one of claims 1 to 6, **characterised in that** it further comprises, after the concentration step (b) and before the fibril-formation step (c), a step of shaping the collagen solution and/or a relaxation step.

8. Method according to claim 7, **characterised in that** the shaping step is a moulding step.

9. Method according to any one of claims 1 to 8, **characterised in that** in step (c), the treatment by a basic gaseous phase is a treatment by ammonia vapour.

10. Method according to any one of claims 1 to 9, **characterised in that** it further comprises, after the fibril-formation step (c), a maturation step.

11. Method for preparing a transparent fibrillated collagen matrix according to any one of claims 1 to 10, comprising the following steps:
(a) preparing an aqueous acid solution comprising:
- collagen, advantageously of type I, possibly associated with other collagen types, at a concentration comprised between 0.1 and 10 mg/ml, advantageously comprised between 0.3 and 5 mg/ml, and
- at least acetic acid and hydrochloric acid.
(b) concentrating the solution obtained in (a) by filtration-centrifugation up to a collagen concentration comprised between 10 and 250 mg/ml, preferably between 30 and 200 mg/ml, and advantageously between 40 and 120 mg/ml;
(c) shaping the collagen solution obtained in (b);
(d) relaxing the collagen solution at the end of step (c);
(e) forming fibrils by treatment of the collagen solution obtained in step (d) by a basic gaseous phase;
(f) maturing fibrils obtained in step (e) until obtaining a matrix.

12. Transparent fibrillated collagen matrix obtainable by the method according to any one of claims 1 to 11, **characterised in that** it transmits at least 80 % of white light.

13. Transparent fibrillated collagen matrix according to claim 12, **characterised in that** its collagen density is equal to or greater than 40 mg/ml.

14. Composite fibrillated collagen matrix obtainable by the method according to any one of claims 1 to 10, **characterised in that** it comprises at least one opaque zone which transmits less than 80 % of white light and at least one transparent zone which transmits at least 80 % of white light.

15. Use of a transparent or composite matrix according to any one of claims 12 to 14, for producing a tissue or an artificial organ, especially a cornea substitute or a cornea-sclera substitute.
